# EUROPÄISCHE PATENTANMELDUNG

(11) **EP 3 020 439 A1**
(43) Veröffentlichungstag der Anmeldung: **18.05.2016**
(21) Anmeldenummer: 15194685.2
(22) Anmeldetag: 16.11.2015
(51) Int. Cl.: A61M 16/00

(54) **BEATMUNGSVORRICHTUNG UND VERFAHREN ZUM BETREIBEN EINER BEATMUNGSVORRICHTUNG**

(30) Priorität: 17.11.2014 DE 102014223396
(71) Anmelder: Seifert, Vera, 64686 Lautertal (DE)
(72) Erfinder: Seifert, Vera, 64686 Lautertal (DE)
(74) Vertreter: Reitstötter Kinzebach

(57) **Zusammenfassung**

Die vorliegende Erfindung betrifft eine Beatmungsvorrichtung (1) umfassend einen Beatmungsbeutel (2) mit wenigstens einem Einlassventil (3) für Sauerstoff-haltige Gase, einem Auslassventil (4) für Sauerstoff-haltige Gase, ein Beatmungselement (5) und eine Betätigungsvorrichtung zur Betätigung des Beatmungsbeutels (2), wobei das Auslassventil (4) für Sauerstoff-haltige Gase zu dem Beatmungselement (5) eine für Sauerstoff-haltige Gase durchströmbare Verbindung aufweist, wobei die Betätigungsvorrichtung eine Fußbedienvorrichtung (10) ist.

## Beschreibung

### HINTERGRUND DER ERFINDUNG

Die vorliegende Erfindung betrifft eine Beatmungsvorrichtung und ein Verfahren zum Bedienen einer Beatmungsvorrichtung.

### STAND DER TECHNIK

Beatmungsvorrichtungen sind im Stand der Technik in einer Vielzahl von Ausführungsformen bekannt. Grundsätzlich kann zwischen automatischen Beatmungsgeräten/ -maschinen und manuell bedienbaren Beatmungsbeuteln unterschieden werden, wobei im Luftsauerstoffkreislauf eines automatischen Beatmungsgerätes meist auch ein manuell bedienbarer Beatmungsbeutel zur spontanen Beatmung oder zum "Anatmen" angeordnet ist.

Beatmungsbeutel werden häufig nach einem bekannten Hersteller auch als Ambu-Beutel bezeichnet. Ein manuell bedienbarer Beatmungsbeutel weist üblicherweise einen selbstexpandierenden Hohlkörper mit einem Lufteinlassventil und einem Luftauslassventil auf. An dem Luftauslassventil wird an einem meist genormten Ansatzstück ein Beatmungselement, z.B. eine Beatmungsmaske oder ein Tubus angebracht. Zur Beatmung wird der Hohlkörper manuell komprimiert, wodurch die sich im Hohlkörper befindende Luft durch das Luftauslassventil durch die Beatmungsmaske oder den Tubus strömt. Sobald der selbstexpandierende Beatmungsbeutel nicht mehr zusammengedrückt wird, wird der Hohlraum des expandierenden Beatmungsbeutels über das Lufteinlassventil mit Luft befüllt und ist bereit für die nächste Beatmungsbeutelkompression. Bei Beatmungsbeuteln, die an automatischen Beatmungsgeräten angeordnet sind, werden die Beatmungsbeutel meist über den im System vorherrschenden Luftdruck befüllt und expandiert.

Ein manuell bedienbarer Beatmungsbeutel ist ein relativ einfaches Hilfsmittel zur Beatmung und zudem nicht auf technische Energiequellen angewiesen, so dass der manuell bedienbare Beatmungsbeutel insbesondere in Notfall-, Transportsituationen und/oder als Reservegerät automatischer Beatmungsgeräte Verwendung findet.

Bekannte Beatmungsvorrichtungen und Verfahren zum Betreiben einer Beatmungsvorrichtung sind noch verbesserungswürdig. So sind bei der Beatmung mit einem manuellen Beatmungsbeutel beide Hände des Bedieners im Einsatz. Mit einer Hand wird der Beatmungsbeutel periodisch komprimiert und mit der anderen Hand wird die Beatmungsmaske mit dem sogenannten C-Griff gehalten. Dabei kann der Bediener des Beatmungsbeutels keine weiteren Maßnahmen oder Bedienungen anderer Vorrichtungen parallel vornehmen.

Der vorliegenden Erfindung liegt daher die Aufgabe zugrunde, eine verbesserte Beatmungsvorrichtung und ein verbessertes Verfahren zum Betreiben der Beatmungsvorrichtung bereitzustellen, bei denen die zuvor genannten Nachteile vermieden werden. Insbesondere soll es mit dieser Beatmungsvorrichtung und dem Verfahren zum Betreiben der Beatmungsvorrichtung möglich sein, beim Bedienen eines Beatmungsbeutels wenigstens eine Hand zur Durchführung anderer, weiterer Maßnahmen zur Verfügung zu haben. Des Weiteren soll die anstrengende, kraftraubende und ermüdende Kompressionsarbeit mit der Hand erleichtert werden. Zudem soll die Möglichkeit bestehen, beim Einsatz eines manuell bedienbaren Beatmungsbeutels die Bedienung einer Beatmungsmaske, insbesondere den passgenauen Sitz der Beatmungsmaske mit beiden Händen zu ermöglichen. Außerdem soll die Beatmungsvorrichtung einfach und platzsparend zu lagern, insbesondere gut stapelbar, einfach und schnell aufzubauen, flexibel einsetzbar, gut desinfizierbar, gut autoklavierbar, zu bereits bestehenden Systemen kompatibel und kostengünstig herstellbar sein.

### ZUSAMMENFASSUNG DER ERFINDUNG

Überraschenderweise wurde nun gefunden, dass diese Aufgabe mit einer Beatmungsvorrichtung umfassend einen Beatmungsbeutel mit wenigstens einem Einlassventil für sauerstoffhaltige Gase, einem Auslassventil für sauerstoffhaltige Gase, ein Beatmungselement und eine Betätigungsvorrichtung zur Betätigung des Beatmungsbeutels, wobei das Auslassventil für sauerstoffhaltige Gase zu dem Beatmungselement eine für sauerstoffhaltige Gase durchströmbare Verbindung aufweist, wobei die Betätigungsvorrichtung eine Fußbedienvorrichtung ist, gelöst wird.

Die erfindungsgemäße Beatmungsvorrichtung weist gegenüber konventionellen manuellen Beatmungsbeuteln den Vorteil auf, dass ihre Betätigung keine manuelle periodische Kompression des Beutels erfordert, so dass wenigstens eine Hand des Bedieners zur Durchführung anderer, weiterer Bedienschritte als der Beatmung zur Verfügung steht. Zudem wird mit der erfindungsgemäßen Beatmungsvorrichtung und dem Beatmungsverfahren die anstrengende, kraftraubende und ermüdende Hand-Kompressionsarbeit bei der Kompression des manuellen Beatmungsbeutels wenigstens teilweise erleichtert. Des Weiteren ist dadurch, dass wenigstens eine Hand zur Durchführung anderer, weiterer Bedienschritte zur Verfügung steht, eine Beatmung stressfreier und eine bessere Planung durchzuführender Arbeitsschritte realisierbar. Vorteilhaft ist, dass die erfindungsgemäße Beatmungsvorrichtung einfach, schnell und leicht bedienbar und kompatibel zu bereits bestehenden Beatmungssystemen ist.

Gegenstand der Erfindung ist daher eine Beatmungsvorrichtung, umfassend
- genau einen Beatmungsbeutel mit wenigstens einem Einlassventil für sauerstoffhaltige Gase und einem Auslassventil für sauerstoffhaltige Gase,
- ein Beatmungselement,
- eine Vorrichtung zur Betätigung des Beatmungsbeutels, und
- eine für sauerstoffhaltige Gase durchströmbare Verbindung zwischen dem Auslassventil für sauerstoffhaltige Gase und dem Beatmungselement,
dadurch gekennzeichnet, das die Vorrichtung zur Betätigung des Beatmungsbeutels eine Fußbedienvorrichtung ist, deren Betätigen eine mechanische Kompression des Beatmungsbeutels bewirkt.

Ein weiterer Gegenstand der Erfindung ist ein Verfahren zum Bedienen einer Beatmungsvorrichtung umfassend die Schritte:
a) Bereitstellen einer Beatmungsvorrichtung umfassend einen Beatmungsbeutel mit wenigstens einem Einlassventil für sauerstoffhaltige Gase und einem Auslassventil für sauerstoffhaltige Gase, ein Beatmungselement, eine Fußbedienvorrichtung zur Betätigung des Beatmungsbeutels und eine für sauerstoffhaltige Gase durchströmbare Verbindung zwischen dem Auslassventil für sauerstoffhaltige Gase und dem Beatmungselement;
b) Anordnen des Beatmungsbeutels, der in der in Schritt a) bereitgestellten Beatmungsvorrichtung umfasst ist, in einem bodennahen Bereich, insbesondere auf einem Fußboden, was eine Fußbedienung, vorzugsweise eine Ein-Fußbedienung des Beatmungsbeutels ermöglicht;
c) Betätigen der Fußbedienvorrichtung, die in der in Schritt a) bereitgestellten Beatmungsvorrichtung umfasst ist.

### DETAILLIERTE BESCHREIBUNG DER ERFINDUNG

Im Rahmen der vorliegenden Erfindung werden unter Sauerstoff-haltigen Gasen Gaszusammensetzungen verstanden, wie sie üblicherweise bei der Beatmung menschlicher Lebewesen eingesetzt werden und die einen zum lebenserhaltenden Atmen ausreichenden hohen Sauerstoffgehalt aufweisen. Insbesondere hat ein Sauerstoff-Stickstoffgemisch einen Volumenanteil an Sauerstoff von mehr als 16 Volumen-% insbesondere wenigstens 25 Volumen-%, vorzugsweise weniger als 1 Volumen-% Kohlendioxid, jeweils bezogen auf das Gesamtvolumen des Sauerstoffhaltiges Gases.

Im Rahmen der vorliegenden Erfindung wird unter einem Beatmungselement eine Vorrichtung verstanden, durch welche die sauerstoffhaltigen Gase aus der Beatmungsvorrichtung in das Atemsystem des zu beatmenden Lebewesens eingeleitet werden. Ein Beatmungselement ist insbesondere eine Beatmungsmaske, ein Tubus, ein Tracheostoma, oder eine Kombination hiervon. Ein Beispiel für eine Beatmungsmaske ist eine Larynxmaske. Beispiele für einen Tubus sind eine Tracheostomiekanüle, ein Endotrachealtubus, ein Larynxtubus und ein Combitubus.

Im Rahmen der vorliegenden Erfindung wird unter einer Fußbedienvorrichtung eine Vorrichtung verstanden, mit der der Beatmungsbeutel komprimiert und gegebenenfalls expandiert werden kann. Die Kompression und gegebenenfalls die Expansion des Beatmungsbeutels erfolgen im Wechsel, insbesondere dem Atemrhythmus entsprechend bzw. diesem angepasst. Durch das Betätigen der erfindungsgemäßen Fußbedienvorrichtung wird eine Kompression, insbesondere eine mechanische Kompression des Beatmungsbeutels bewirkt. Durch Nicht-Betätigen der Fußbedienvorrichtung wird der Beatmungsbeutel in einer bevorzugten Ausgestaltung expandiert.

Die Fußbedienvorrichtung ist insbesondere als Pedal ausgestaltet. Unter einem Pedal wird eine mechanische Vorrichtung verstanden, die mit dem Fuß zu bedienen ist und zur Kraftübertragung von einem oder mehreren Füßen genutzt wird. Das Pedal kann als Hebel, Taster, Schalter, Wippe, eine Presse, Zange oder beliebigen Kombinationen hiervon ausgestaltet sein. Auch ist es möglich, die Fußbedienvorrichtung und den Beatmungsbeutel als Blasebalg auszugestalten. Beim Bedienen aller vorgenannten Ausführungsformen der Fußbedienvorrichtung wird eine geradlinige oder bogenförmige (Hub-)Bewegung mit dem Fuß durchgeführt.

Die Fußbedienvorrichtung ist vorzugsweise in einem bodennahen Bereich angeordnet. Hierunter wird ein Bereich verstanden, welcher bei einer Fußbedienung für wenigstens einen von zwei Füßen erreichbar ist, so dass eine Betätigung mit einem Fuß durchgeführt werden kann.

In einer besonders bevorzugten Ausführungsform der Erfindung ist die Fußbedienvorrichtung als Pedal ausgestaltet, das im Wesentlichen ein Bodengestell, einen beweglichen Hebel und ein Widerlager umfasst. Das Bodengestell kann ein flächiges festes Gebilde, wie beispielsweise eine Platte, oder ein rahmenartiges steifes Gebilde, wie beispielsweise ein Rahmen aus Latten oder Vierkantträger in runder, ovaler, U-, V- oder Polygon-Form, sein.

Der Beatmungsbeutel wird zwischen dem Bodengestell und dem bewegten Teil eingebracht und gegebenenfalls befestigt und/oder fixiert. Bei Betätigen des Pedals bewegt sich der bewegliche Hebel gegen das am Bodengestell befestigte Widerlager. Der zwischen dem Boden(-Gestell) und dem bewegten Teil befindliche Beatmungsbeutel wird durch den beweglichen Hebel gegen den/das Boden(-Gestell) gepresst und dabei komprimiert. In der Regel entspricht die Bewegung des beweglichen Teils einer geradlinigen oder bogenförmigen Bewegung.

In einer bevorzugten Ausführungsform der Erfindung ist an der Fußbedienvorrichtung eine Expansionsvorrichtung angeordnet, die eine Expansion des Beatmungsbeutels bei Nicht-Betätigen der Fußbedienvorrichtung bewirkt. Über die Expansionsvorrichtung kann der Beatmungsbeutel nach erfolgter Kompression wieder expandiert werden. Die Wirkrichtung der Expansion ist entgegengesetzt zu der Kraftwirkung bei der Kompression. Die Expansionsvorrichtung kann ausgewählt sein unter mechanischen, pneumatischen und hydraulischen Ausdehnvorrichtungen sowie beliebigen Kombinationen hiervon.

Darüber hinaus kann das Pedal noch mit einem oder mehreren Befestigungselementen und/oder einem oder mehreren Fixierelementen ausgestattet sein. Mit den Befestigungselementen und/oder Fixierelementen kann der Beatmungsbeutel vorübergehend oder dauerhaft in der Beatmungsvorrichtung im Bereich der Fußbedienvorrichtung angeordnet werden. Durch die Befestigungs- und/oder Fixierelemente kann die Kraftübertragung der Fußbedienvorrichtung auf den Beatmungsbeutel optimiert werden.

In einer bevorzugten Ausführungsform der Erfindung ist das wenigstens eine Einlassventil mit dem einen Auslassventil strömungstechnisch kombiniert als ein Ein-/Auslassventil und als ein Zwei- oder Drei-Wege-Ventil ausgestaltet. In einer besonders bevorzugten Ausgestaltung handelt es sich bei dem Ein-/Auslassventil um ein 3/2-Wege-Ventil.

Ein Zwei-Wege-Ventil weist in Rahmen der vorliegenden Erfindung einen gemeinsamen Durchflussweg zum Einströmen und zum Ausströmen der sauerstoffhaltigen Gase auf, wobei sich die Strömungsrichtung beim Ausströmen gegenüber dem Einströmen umkehrt. Insbesondere ist das strömungstechnisch kombinierte Zwei-Wege-Ventil derart ausgestaltet, dass für den Durchflussweg zum Ein- und Ausströmen der sauerstoffhaltige Gase ein technisch gemeinsam wirkendes Ventilsteuer-element angeordnet ist, das wechselseitig die erste Durchflussrichtung oder die zweite Durchflussrichtung wenigstens teilweise freigibt und jeweils die andere Durchflussrichtung versperrt. Ein Drei-Wege-Ventil hingegen weist im Rahmen der vorliegenden Erfindung mindestens einen ersten Durchflussweg zum Einströmen der sauerstoffhaltigen Gase und einen zweiten Durchflussweg zum Ausströmen der sauerstoffhaltigen Gase auf. Insbesondere ist das Drei-Wege-Ventil derart ausgestaltet, dass für den ersten Durchflussweg zum Einströmen und den zweiten Durchflussweg zum Ausströmen der sauerstoffhaltigen Gase ein für beide Durchflusswege technisch gemeinsam wirkendes Ventilsteuerelement angeordnet ist, das wechselseitig den ersten Durchflussweg oder den zweiten Durchflussweg wenigstens teilweise freigibt und jeweils den anderen Durchflussweg versperrt. Im Gegensatz zu einem separaten Einlassventil und einem separaten Auslassventil ist bei einem kombinierten Ein-/Auslassventil nur eine Öffnung im Beatmungsbeutel angeordnet.

In einer besonders bevorzugten Ausführungsform der erfindungsgemäßen Vorrichtung ist der Beatmungsbeutel im Wesentlichen ovoid. Unter im Wesentlichen ovoid wird im Sinne der vorliegenden Erfindung verstanden, dass der betrachtete Körper in seiner Neutralform eine rundliche konvexe Form aufweist. Die Neutralform bezeichnet hier und im Folgenden die nicht komprimierte Form, also die Form des Beatmungsbeutels im vollständig befüllten Zustand mit Gas bei Umgebungsdruck oder wenig darüber. Die Form des Beatmungsbeutels ist dabei nicht auf Kugeln oder Ellipsoide beschränkt, sondern umfasst jede dreidimensionale konvexe Form, die sich aus rotierten Kreisbögen und/oder Geradenstücken zusammensetzen lässt. Ventile und/oder Anschlussöffnungen sowie gegebenenfalls an der Außenseite des Beatmungsbeutels angebrachte Fixier-, Befestigungs- oder Halteeinrichtungen bleiben bei der Betrachtung der Form des Beatmungsbeutels unberücksichtigt.

In einer bevorzugten Ausführungsform der Erfindung ist der Beatmungsbeutel selbstexpandierend. Unter einem selbstexpandierenden Beatmungsbeutel versteht man, dass sich der Beatmungsbeutel nach erfolgter Kompression selbsttätig in seine nicht komprimierte Form, hier und im Folgenden auch als Neutralform bezeichnet, ausdehnt. Dies wird üblicherweise auf mechanischem Wege erreicht. Dazu ist der Beatmungsbeutel mit mechanischen Einrichtungen und/oder Hilfsmitteln versehen, die ihn ohne von außen einwirkende (Druck-)Kräfte in seine Neutralform zurückbringen Insbesondere werden zu diesem Zweck Elemente verwendet, die sich elastisch verformen lassen und dabei wenigstens einen Teil der verrichteten Formänderungsarbeit als potentielle Energie speichern. Geeignete Beispiele sind Federelemente, Spannelemente, Dämpfungselemente, Dehnelemente und beliebige Kombinationen hiervon. Eine weitere Möglichkeit ist eine pneumatische Ausdehnung. Bei der pneumatischen Ausdehnung ist beispielsweise der in dem Kreislauf der Beatmungsvorrichtung vorherrschende Druck des sauerstoffhaltigen Gases für die Expansion des Beatmungsbeutels verantwortlich. Prinzipiell ist auch die Verwendung von Beatmungsbeuteln möglich, die nach dem Prinzip eines Faltenbalgs arbeiten. Aufgrund der hygienischen Anforderungen, insbesondere an Reinigung, Desinfektion oder Sterilisation, werden aber bevorzugt selbstexpandierende ovoide Beatmungsbeutel verwendet.

Erfindungsgemäß können wiederverwendbare oder Einmal-Beatmungsbeutel eingesetzt werden. Für den Anwendungsfall geeignete Materialien sind dem Fachmann hinlänglich bekannt, beispielsweise Silikon, Latex, PVC. Erfindungsgemäß verwendete Beatmungsbeutel sind kommerziell erhältlich, beispielsweise unter den Markennamen Ambu®-Beutel oder SilkoBag von Rüsch, sowie von Anbietern wie beispielsweise Laerdal, Weinmann, etc.

In einer bevorzugten Ausführungsform der Erfindung weist der Beatmungsbeutel nach einer Kompression bis zur vollständigen Expansion eine Expansionszeit im Bereich von 1 s bis 10 s, vorzugsweise im Bereich von 1 s bis 6 s, besonders bevorzugt im Bereich von 1 s bis 4 s, ganz besonders bevorzugt im Bereich von 1 s bis 2 s auf. Die Expansionszeit hat einen Einfluss auf die von dem Beatmungsbeutel zur Verfügung gestellte theoretisch mögliche Beatmungsfrequenz beziehungsweise der möglichen Komprimierungen des Beatmungsbeutels pro Zeiteinheit. Bei kurzen Expansionszeiten können höhere Beatmungsfrequenzen, das heißt eine größere Anzahl an Kompressionen durchgeführt werden als bei längeren Expansionszeiten des Beatmungsbeutels.

Die hier angegebenen Zeiten beziehen sich auf das Zeitintervall, welches der Beatmungsbeutel benötigt von einem weitgehend komprimierten Zustand, in welchem das Innenvolumen des Beatmungsbeutels auf einen Volumenanteil von mehr als 70 % des Gesamtvolumens reduziert ist, sich vollständig zu entspannen.

In einer bevorzugten Ausführungsform der Erfindung ist die Sauerstoffgemisch durchströmbare Verbindung zwischen dem Beatmungsbeutel und dem Beatmungselement wenigstens ein flexibler Verbindungsschlauch mit einer Länge im Bereich von 80 cm bis 300 cm, vorzugsweise im Bereich von 80 cm bis 250 cm, besonders bevorzugt im Bereich von 80 cm bis 200 cm, ganz besonders bevorzugt im Bereich von 80 cm bis 180 cm.

In einer bevorzugten Ausführungsform der Erfindung ist der wenigstens eine flexible Verbindungsschlauch zwischen dem Beatmungsbeutel und dem Beatmungselement mit mindestens einer lösbaren Verbindung angeordnet. Unter einer lösbaren Verbindung werden Verbindungen verstanden, welche sich durch Umkehr des Verbindungsvorganges wieder trennen lassen. Eine lösbare Verbindung kann insbesondere ausgewählt werden unter einer Steckverbindung, einer Rastverbindung einer Kupplungsverbindung, einer Drehschlussverbindung, einer Klemmverbindung, einer Spannverbindung und Kombinationen hiervon. Die lösbare Verbindung ist insbesondere eine Steckverbindung mit einer Klemmkraftwirkung. Die lösbare Verbindung kann von einem Fluid, insbesondere von sauerstoffhaltigen Gasen durchströmt werden.

In einer weiteren bevorzugten Ausführungsform der Erfindung ist der Beatmungsbeutel in der Fußbedienvorrichtung lösbar angeordnet. Die lösbare Anordnung des Beatmungsbeutels ermöglicht, dass der Beatmungsbeutel bei Bedarf aus der Fußbedienvorrichtung heraus genommen werden kann und der Beatmungsbeutel beispielsweise manuell komprimiert werden kann.

In einer geeigneten Ausführungsform weist der Beatmungsbeutel wenigstens ein Fixierelement für eine stationäre wiederlösbare Fixierung des Beatmungsbeutels an der Fußbedienvorrichtung und/oder auf einem festen Untergrund, insbesondere auf einem Fußboden auf. Mit einem Fixierelement kann der Beatmungsbeutel vorübergehend oder dauerhaft an der Fußbedienvorrichtung, einem geeigneten Untergrund, wie beispielsweise dem Fußboden, fixiert werden.

In einer geeigneten Ausführungsform der Erfindung ist am Beatmungsbeutel eine Expansionsvorrichtung vorgesehen, die den Beatmungsbeutel nach seiner Kompression wieder expandiert. Bei der Expansionsvorrichtung ist die Wirkrichtung der Expansion des Beatmungsbeutels entgegengesetzt zu derjenigen bei der Kraftwirkung bei seiner Komprimierung. Die Expansionsvorrichtung kann ausgewählt sein unter mechanischen, pneumatischen, hydraulischen Ausdehnvorrichtungen und Kombinationen hiervon.

In einer geeigneten alternativen Ausgestaltung kann die erfindungsgemäße Beatmungsvorrichtung auch an ein Atemhilfsgerät angeschlossen werden. Das Atemhilfsgerät dient dann zur Bereitstellung von Atemluft. Atemhilfsgeräte sind dem Fachmann hinlänglich bekannt, beispielsweise als konventionelle Beatmungsmaschine. Wird zusätzlich ein Atemhilfsgerät verwendet, hat es sich als vorteilhaft erwiesen, das Beatmungselement, beispielsweise einen Tubus oder eine Maske, über einen Patientenschlauch bzw. ein Patientenschlauchsystem an das Atemhilfsgerät, beispielsweise die Beatmungsmaschine, anzuschließen. Das Anschließen an ein Atemhilfsgerät kann besonders nach erfolgter Erstversorgung des Patienten vorteilhaft sein.

In einer ebenfalls geeigneten alternativen Ausgestaltung kann zusätzlich ein Luftreservoirbeutel oder ein anderes geeignetes, handelsübliches Sauerstoffreservoir an den Beatmungsbeutel angeschlossen werden.

Gegenstand der vorliegenden Erfindung ist auch ein Verfahren zum Bedienen einer erfindungsgemäßen Beatmungsvorrichtung, das folgende Schritte umfasst:
a) Bereitstellen einer erfindungsgemäßen Beatmungsvorrichtung;
b) Anordnen des Beatmungsbeutels, der in der in Schritt a) bereitgestellten Beatmungsvorrichtung umfasst ist, in einem bodennahen Bereich, insbesondere auf einem Fußboden;
c) Betätigen der Fußbedienvorrichtung, die in der in Schritt a) bereitgestellten Beatmungsvorrichtung umfasst ist,
wobei während, zwischen oder nach den Schritten a) und b) das Beatmungselement an einer zu beatmenden Person platziert wird.

In einer bevorzugten Ausgestaltung des erfindungsgemäßen Verfahrens wird in Schritt a) die Beatmungsvorrichtung aus den Komponenten Beatmungsbeutel, Beatmungselement, durchströmbare Verbindung und Fußbedienvorrichtung bereits vorinstalliert bereitgestellt.

In einer ebenfalls bevorzugten Ausgestaltung des erfindungsgemäßen Verfahrens umfasst Schritt a) das Montieren des Beatmungsbeutels, des Beatmungselementes und der durchströmbaren Verbindung. In Schritt b) wird dann der Beatmungsbeutel in der Fußbedienvorrichtung angeordnet und befestigt.

In einer weiteren bevorzugten Ausgestaltung des erfindungsgemäßen Verfahrens erfolgt in Schritt c) die Fußbedienung als Ein-Fußbedienung des Beatmungsbeutels in der Beatmungsvorrichtung.

### FIGURENBESCHREIBUNG UND BEISPIELE

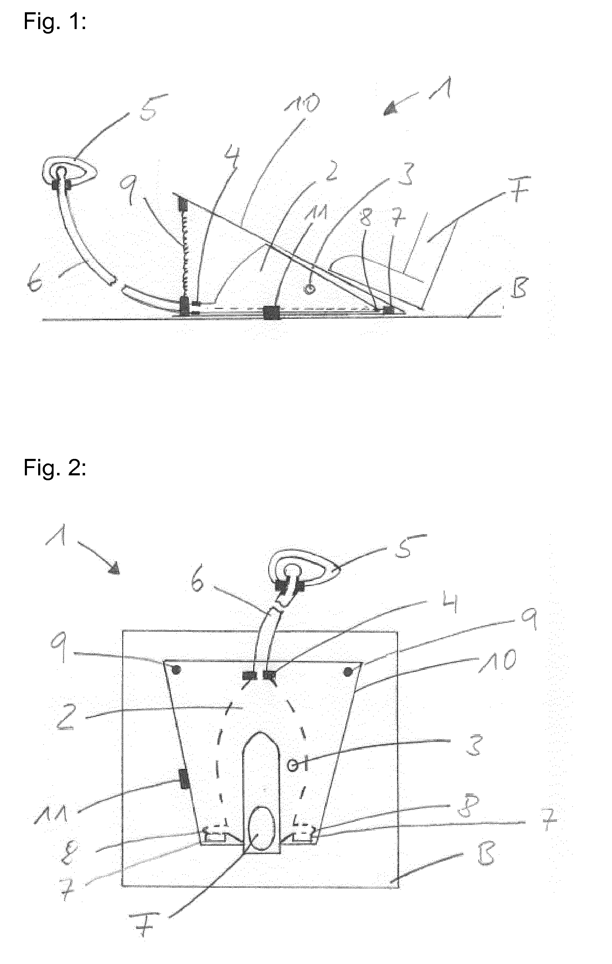

Die Erfindung wird nun anhand der Figuren 1 und 2 und des Ausführungsbeispiels 1 nachfolgend erläutert.
- Fig. 1: zeigt schematisch einen Querschnitt einer Seitenansicht der Beatmungsvorrichtung gemäß einer Ausführungsform der Erfindung mit einer Fußbedienvorrichtung.
- Fig. 2: zeigt schematisch eine Aufsicht der Beatmungsvorrichtung gemäß einer Ausführungsform der Erfindung mit einer Fußbedienvorrichtung nach Figur 1.
- Fig. 3: zeigt schematisch einen Querschnitt einer bevorzugten Ausführungsform der erfindungsgemäßen Beatmungsvorrichtung.

In Figur 1 ist eine Ausführungsform der erfindungsgemäßen Beatmungsvorrichtung 1 schematisch als ein Querschnitt einer Seitenansicht dargestellt. Ein Beatmungsbeutel 2 ist auf einem (Fuß-)Boden B liegend dargestellt. An dem Beatmungsbeutel 2 sind ein Einlassventil 3 für sauerstoffhaltige Gase und ein Auslassventil 4 für sauerstoffhaltige Gase angeordnet. Das Auslassventil 4 ist mit einem Verbindungsschlauch 6 mit einem Beatmungselement 5 verbunden. Oberhalb des Beatmungsbeutels 2 ist ein Fuß F zur Fußbedienung der Beatmungsvorrichtung 1 dargestellt. Der Beatmungsbeutel 2 ist in einer Fußbedienvorrichtung 10 angeordnet und kann mit wenigstens einem Fixierelement 8 des Beatmungsbeutels 2 an wenigstens einem Befestigungselement 7 der Fußbedienvorrichtung 10 lösbar befestigt werden. Die Fußbedienvorrichtung 10 weist eine Expansionsvorrichtung 9, beispielhaft dargestellt als eine Rückholfeder, auf. Der an dem Auslassventil 4 angebrachte Verbindungsschlauch 6 führt an der Expansionsvorrichtung 9 vorbei zu dem Beatmungselement 5. An der Fußbedienvorrichtung 10 ist ein Auslöseschalter 11 angeordnet. Bei Betätigung des Auslöseschalters 11 löst sich die Verbindung zwischen dem wenigstens einem Befestigungselement 7 und dem wenigstens einem Fixierelement 8 und der Beatmungsbeutel 2 kann aus der Fußbedienvorrichtung 10 entnommen werden und beispielsweise manuell bedient werden.

Figur 2 zeigt schematisch eine Aufsicht der Beatmungsvorrichtung 1 gemäß einer Ausführungsform der Erfindung mit der Fußbedienvorrichtung 10 nach Figur 1. Die Beatmungsvorrichtung 10 ist auf dem (Fuß-)Boden B liegend angeordnet und mit dem Fuß F zur Fußbedienung dargestellt. Innerhalb der Fußbedienvorrichtung 10 ist der Beatmungsbeutel 2 (gestrichelt dargestellt) mit zwei Fixierelementen 8, einem Einlassventil 3 für sauerstoffhaltige Gase und einem Auslassventil 4 für sauerstoffhaltige Gase angeordnet. Der an dem Auslassventil 4 angebrachte Verbindungsschlauch 6 führt zu einem Beatmungselement 5.

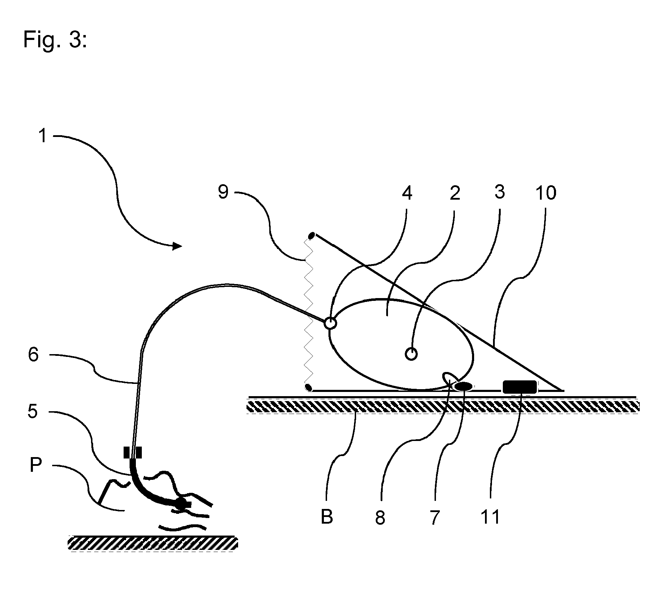

Figur 3 zeigt schematisch eine besonders bevorzugte Ausführungsform der erfindungsgemäßen Beatmungsvorrichtung 1 im Querschnitt. Ein ovoider Beatmungsbeutel 2 mit einem Einlassventil 3 und einem Auslassventil 4 ist in einer Fußbedienvorrichtung 10 angeordnet. Der Beatmungsbeutel 2 ist mittels des Auslassventils 4 über einen Verbindungsschlauch 6 mit einem Beatmungselement 5 verbunden. Das Beatmungselement 5 ist, beispielsweise ein Endotrachealtubus, in der Luftröhre einer zu beatmenden Person P platziert. Der Beatmungsbeutel 2 kann mittels eines Fixierelementes 8 lösbar an einem Befestigungselement 7 der Fußbedienvorrichtung 10 befestigt werden. Die Fußbedienvorrichtung 10 weist weiterhin eine Expansionsvorrichtung 9, hier als Feder dargestellt, sowie einen Auslöseschalter 11 auf. Bei Betätigen des Auslöseschalters 11 löst sich die Verbindung zwischen Befestigungselement 7 und Fixierelement 8, so dass der Beatmungsbeutel 2 aus der Fußbedienvorrichtung 10 entnommen werden kann. Der Beatmungsbeutel 2 kann dann alternativ manuell bedient oder an ein geeignetes Atemhilfsgerät, wie beispielsweise eine konventionelle Beatmungsmaschine, angeschlossen werden.

### Bezugszeichenliste:

- 1:: Beatmungsvorrichtung
- 2:: Beatmungsbeutel
- 3:: Einlassventil für sauerstoffhaltige Gase
- 4:: Auslassventil für sauerstoffhaltige Gase
- 5:: Beatmungselement
- 6:: Verbindungsschlauch
- 7:: Befestigungselement
- 8:: Fixierelement
- 9:: Expansionsvorrichtung
- 10:: Fußbedienvorrichtung
- 11:: Auslöseschalter
- B:: (Fuß-)Boden
- F:: Fuß
- P:: (zu beatmende) Person

### Beispiel 1

Die Beatmungsvorrichtung wurde wie in den Figuren 1 bis 2 dargestellt gefertigt. Als Beatmungsbeutel kam der Ambu-Beutel des Unternehmens Ambu A/S oder der Silko Bag des Unternehmens Rüsch zum Einsatz. Der Verbindungsschlauch kam mit den Schlauchlängen 80 cm, 150 cm, 200 cm, 250 cm und 300 cm zum Einsatz. Als Fußbedienvorrichtung wurden zwei Holzplatten als Schenkel mit einem im spitzen Winkel angeordneten Scharnier verbunden und der Beatmungsbeutel mit seinen Ösen ausgeführt als Fixierelementen an den Befestigungshaken ausgeführt als Befestigungselemente der Fußbedienvorrichtung lösbar eingehakt. Als Expansionsvorrichtung wurde an der dem spitzen Winkel gegenüber liegenden Seite eine Rückstellfeder angeordnet.

## Patentansprüche

1. Beatmungsvorrichtung umfassend
- genau einen Beatmungsbeutel mit wenigstens einem Einlassventil für sauerstoffhaltige Gase und einem Auslassventil für sauerstoffhaltige Gase,
- ein Beatmungselement,
- eine Vorrichtung zur Betätigung des Beatmungsbeutels, und
- eine für sauerstoffhaltige Gase durchströmbare Verbindung zwischen dem Auslassventil für sauerstoffhaltige Gase und dem Beatmungselement,
**dadurch gekennzeichnet, dass** die Vorrichtung zur Betätigung des Beatmungsbeutels eine Fußbedienvorrichtung ist, deren Betätigen eine Kompression des Beatmungsbeutels bewirkt.

2. Beatmungsvorrichtung nach Anspruch 1, wobei die Fußbedienvorrichtung als Pedal ausgestaltet ist.

3. Beatmungsvorrichtung nach einem der vorherigen Ansprüche, wobei am Beatmungsbeutel das Einlassventil mit dem Auslassventil strömungstechnisch kombiniert und als Zwei- oder Drei- Wege-Ventil ausgestaltet ist.

4. Beatmungsvorrichtung nach einem der vorherigen Ansprüche, wobei der Beatmungsbeutel im Wesentlichen ovoid ist.

5. Beatmungsvorrichtung nach einem der vorherigen Ansprüche, wobei der Beatmungsbeutel selbstexpandierend ist.

6. Beatmungsvorrichtung nach einem der vorherigen Ansprüche, wobei die durchströmbare Verbindung zwischen dem Beatmungsbeutel und dem Beatmungselement ein flexibler Verbindungsschlauch mit einer Länge im Bereich von 80 cm bis 300 cm ist, der zwischen dem Beatmungsbeutel und dem Beatmungselement mit mindestens einer lösbaren Verbindung angeordnet sein kann.

7. Beatmungsvorrichtung nach einem der vorherigen Ansprüche, wobei der Beatmungsbeutel in der Fußbedienvorrichtung und/oder auf einem festen Untergrund, insbesondere auf einem Fußboden, lösbar und/oder mittels eines oder mehrerer am Beatmungsbeutel angeordneten Fixierelementen und/oder eines oder mehrerer an der Fußbedienvorrichtung angeordneten Befestigungselementen angeordnet ist.

8. Beatmungsvorrichtung nach einem der vorherigen Ansprüche, wobei die Fußbedienvorrichtung eine Expansionsvorrichtung aufweist, die eine Expansion des Beatmungsbeutels bei Nicht-Betätigen der Fußbedienvorrichtung bewirkt.

9. Verfahren zum Bedienen einer Beatmungsvorrichtung umfassend die Schritte:
a) Bereitstellen einer Beatmungsvorrichtung gemäß einem der Ansprüche 1 bis 8;
b) Anordnen des Beatmungsbeutels, der in der in Schritt a) bereitgestellten Beatmungsvorrichtung umfasst ist, in einem bodennahen Bereich, insbesondere auf einem Fußboden;
c) Betätigen der Fußbedienvorrichtung, die in der in Schritt a) bereitgestellten Beatmungsvorrichtung umfasst ist,
wobei während, zwischen oder nach den Schritten a) und b) das Beatmungselement an einer zu beatmenden Person platziert wird.

10. Verfahren gemäß Anspruch 9, das wenigstens eines der beiden folgenden Merkmale aufweist:
- entweder wird in Schritt a) die Beatmungsvorrichtung aus den Komponenten Beatmungsbeutel, Beatmungselement, durchströmbare Verbindung und Fußbedienvorrichtung bereits vorinstalliert bereitgestellt, oder in Schritt a) erfolgt das Montieren des Beatmungsbeutels, des Beatmungselementes und der durchströmbaren Verbindung und in Schritt b) wird dann der Beatmungsbeutel in der Fußbedienvorrichtung angeordnet und befestigt;
- in Schritt c) erfolgt die Fußbedienung als Ein-Fußbedienung des Beatmungsbeutels in der Beatmungsvorrichtung.
